# EUROPEAN PATENT APPLICATION

(11) **EP 2 286 842 A1**
(43) Date of publication of application: **23.02.2011**
(21) Application number: 08761557.1
(22) Date of filing: 06.05.2008
(51) Int. Cl.: A61K 47/44, A61Q 11/00

(54) **TOOTH MOVEMENT ACTIVATING COMPOUND**

(71) Applicant: Instituto de Investigación en Ortodoncia, S.L., 28016 Madrid (ES)
(72) Inventor: ALIÓ SANZ, Juan José, 28002 Madrid (ES)
(74) Representative: Mato Adrover, Ángel Luis
(86) International application number: PCT/ES2008/000312
(87) International publication number: WO 2009/135957

(57) **Abstract**

The invention relates to the development of a food or edible product of oleic origin, which acts as a dental lubricant and is intended to reduce friction between orthodontic wires and brackets, thereby providing low, more continuous physiological forces and, consequently, a significant reduction in orthodontic treatment times. The invention is **characterised in that** it comprises a base element of oleic origin and a stabilising or mixing element and in that it is prepared so that it can also include different additives having sanitary-hygienic properties. The invention is also **characterised in that** it can be applied easily and immediately and in that it is formulated with different viscosities in order to cover all orthodontic treatment phases.

## Description

### Object of the Invention

The present invention is aimed at defining a new food compound derived from oleic type fatty acids to reduce friction or frictional force between orthodontic wires and brackets, thereby preventing unwanted force components.

Lower friction means low forces, and it has been proved that by reducing the friction a biologically compatible tooth movement and a significant reduction (up to 60%) of the duration of the orthodontic treatment are possible.

Low forces throughout the entire orthodontic treatment also mean greater comfort for the patient.

### Background of the Invention

Orthodontics is the specialty of dentistry that is concerned with the movement, position and development of the masticatory apparatus. It also relates to the science in charge of the study, prevention, diagnosis and treatment of dentomaxillofacial structure anomalies.

The technological progress in this specialty of dentistry is fundamentally aimed at solving the main needs put forth by society in this science field, such as achieving a comfortable and long-lasting dentition, suitable aesthetics, the reduction of discomfort in the patient, shorter duration of the orthodontic treatment and lower cost thereof.

Most of the aforementioned needs are being solved by reducing the bracket-wire friction and therefore minimizing the negative effect of force components.

One of the methods has been to use conventional surface chemistry techniques, such as the implantation of ions, plasma and carbon, although up until now it has not been possible to introduce it into clinical practice, fundamentally due to the low persistence thereof in the mouth, doubtful result and high cost.

Another route undertaken to reduce this bracket-wire friction is to study the physicochemical parameters affecting the structural morphology and composition of the elements involved in the orthodontic treatment, such as the study of the roughness of the surfaces in contact, the rigidity of the wire and its hardness, studying the different coefficients of friction between the various combinations of the acting elements. Thus, different alloys for manufacturing wires are being made, such as alloys of austenitic refractory stainless steel alloyed with chromium-nickel and low carbon content, austenitic ferritic stainless steel alloyed with chromium, molybdenum and low nickel content, cobalt-chromium alloys (the latter are mainly of cobalt with significant amounts of chromium to improve the corrosion resistance), cobalt-chromium-molybdenum alloys, titanium alloys, widely used due to their exceptional biocompatibility and corrosion resistance, furthermore, the surfaces of titanium oxide and the alloys thereof are well tolerated in contact with bone, β-titanium alloys, these alloys have concentrations of molybdenum greater than 10%, to stabilize the β phase at room temperature.

The design, conformation and composition of bracket materials are also being researched for the purpose, as has been stated, of reducing the coefficient of friction.

In relation to the design, increasingly smaller brackets are being manufactured, whereby the surface of contact between the latter and the orthodontic wire is reduced, and therefore friction is reduced, brackets with bevelled angles in the channel where the orthodontic wire is housed, also to reduce the roughness of the walls of the bracket, and therefore the friction, are also being manufactured with injection-mouldable materials.

Low-friction self-ligating brackets are currently being developed.

The research of bracket materials for reducing friction has taken placed in parallel with that of orthodontic wires.

Brackets are traditionally made of stainless steel, alloys of this material with titanium, but the latter are relatively soft and therefore susceptible to wear and rubbing on the surface of contact with the wire and consequently cause abrasion and release of toxic materials. Furthermore, they have the drawback that, since the orthodontic wires are made of stainless steel alloys containing chromium and nickel or titanium and nickel alloys, both chromium and nickel being toxic, they may be carcinogenic. In order to partly prevent this abrasion, layers of titanium, zirconium or hafnium compounds are incorporated.

Low-friction ligatures consisting of mixtures of medical use polyurethane are also being developed.

In addition, lubrication assays with elastic ligatures and a search for efficient lubricants, up until now with an aqueous or alcoholic base, are being conducted.

A clear example of the latter is patent US2004101489, which uses a polyvinyl alcohol polymer, or other patents which use water as the base element base and which are dental treatments rather than dental lubricants.

The patent project object of this specification attempts to introduce a new variable which has not been used up until now in the orthodontics field, such as the use of lubricants of oleic origin applied on dental apparatuses to reduce friction and achieve more continuous physiological forces, and which shortens certain orthodontic treatment phases.

A lubricant is understood as a substance which, when placed between two mobile parts, forms a film preventing the contact thereof, thereby preventing the friction or appearance of unwanted force components and therefore a more physiological tooth movement.

The synthesized compound has been proved as non-toxic and as a food compound and, as indicated in the title, it is referred to as tooth movement activating compound.

The technical problem solved by this patent is the reduction of friction between the walls of the bracket and the orthodontic wire by means of a food or edible compound which is easy to apply, has consistency and is long-lasting.

The persistence and stability of the tooth movement activating compound have been proved.

In orthodontic treatment, the section of the orthodontic wire is normally varied, having a smaller section in the first phases and it is gradually increased as the treatment progresses. In this respect, the tooth movement activating compound can act in any phase since there are different degrees of density.

Tribological studies demonstrate that a reduction of friction by 54%, using wires made of stainless steel alloyed with titanium, and by 61% with wires made of stainless steel, shorten the levelling period by approximately eight months, whereby the total reduction of the orthodontic treatment can be 45%.

Practical preliminary studies confirm the clinical usefulness, verifying significant differences between the groups of patients who are treated with the tooth movement activating compound and those who are not.

### Description of the Invention

The work consisted of synthesizing a new product from food or edible fats or oils used as a base, a stabilising or mixing compound and additives for different purposes or functions for the application thereof as a lubricant in the orthodontic treatment with fixed apparatuses for maximum tooth control, for which it was necessary to take comparative tribological measurements on model systems and materials.

Food or edible fats and oils are understood as those which are included in the definition of the Codex Committee on Fats and Oils, i.e., those which are made up of fatty acid glycerides and are of a plant, animal, including milk, or marine origin such as castor oil, coconut oil, cottonseed oil, fish oil, grape seed oil, peanut oil, hydrogenated oils, linseed oil, corn oil, olive oil, olive pomace oil, palm oil, palm olein, palm stearin, palm kernel oil, rapeseed oil, safflower oil, sesame oil, sunflower oil, soybean oil, apricot kernel oil, babassu oil, mustard seed oil, turnip rape or ravison oil, lard, stearin, illipe butter, shea butter, tallow, synthetic edible fats or oils, liquid petroleum jelly, glycerol, the mixture thereof and also the fats and oils which have been subjected to modification treatments, such as transesterification or hydrogenation or fractionation and those which must be subjected to a subsequent preparation in order to make them suitable for human consumption and food or edible synthetic fats and oils.

Polyglycosylated saturated hydrogenated palm oil glycerides, polyethylene glycol esters of apricot kernel oils or polyethylene glycol stearates are used as a stabilising or mixing element.

The additives in the formulation of the tooth movement activating compound allow different compounds having the properties of: antiseptic agents such as triclosan, chlorophenylbiguanidine and derivatives, such as chlorhexidine, chlorhexidine digluconate, benzalkonium chloride, povidone-iodine molecular complex, mercurial antiseptic, merbromin, cetylpyridinium chloride, mouthwashes with hexidine, choline hexasalicylate, cetalkonium chloride, calcium carbonate, sodium citrate, dimethicone, nicomethanol hydrofluoride, 2^{nd} generation fluoroamines, ethanol, propanol, benzoic chloride. Bactericides, for germs and bacteriostatic agents, potassium nitrate, xylitol, preparations with benzydamine HCl, chlorhexidine digluconate, hexetidine, Oralkin hexetidine. Fungicides such as, in addition to some of those mentioned above which have this property, sodium hypochlorite, acetic acid, alkaline peroxides, chlorhexidine gluconate. Antiviral agents, aloe vera, antiprotozoal agents, sporicides, anti-inflammatory agents, mucosal protectors, flavouring agents, antiplaque agents, such as zinc acetate, cetylpyridinium chloride, zinc lactate, fluorine derivatives such as sodium fluoride, sodium monofluorophosphate. Antihalitosis agents, such as triclosan, zinc chloride, cetylpyridinium chloride, zinc lactate. Anticavity agents. Most of these substances usually have more than one of the mentioned properties.

Once the initial objective was fulfilled, the successive research steps which completed the development of the product were conducted, and the clinical efficacy thereof was verified.

The assays have been extended to studying the influence of the application of amorphous carbon coatings in combination with the lubricant of oleic origin.

"In vitro" studies demonstrating their biomechanical usefulness are still being conducted, for which a numerical finite-element biomechanical device or model is being used.

Randomized clinical trials on patients have also been conducted.

The tooth movement activating compound object of this specification is approved by all the official organisms as a non-toxic food compound.

The project was completed with a tribological study, with which the coefficients of friction between different combinations of materials in the presence or absence of lubricants and always under physiological conditions were measured. Studies which determined the influence of surface polishing treatments and the application of lubricant carbon coatings on brackets and wires were also conducted.

Friction, as has been indicated, is a highly determining factor in the efficacy of an orthodontic apparatus. Friction increases the force required to move a tooth, slows down tooth movement and contributes to the anchorage loss.

Several physical and biological factors are involved in friction in orthodontics, the properties of the bracket, (material, manufacturing process, design), properties of the wire, (material and cross-section), ligating method, factors of the patient (bracket-wire angulation, intra-oral dynamic forces) and biological films.

Studies have demonstrated that the greater the contact between the wire and the bracket, the greater the friction.

The effect of the angulation in friction is more pronounced with dental arches or wires made of stainless steel in comparison with those made of nickel-titanium. This can be explained by the lower rigidity of the Ni-Ti wires.

The increased friction due to a greater angulation of the bracket is frequently attributed to the fact that the wires bend.

There is a continuous interest in improving the aesthetics of orthodontic apparatuses, such as ceramic brackets and the reduction of friction is one of the most important goals for the new generation of ceramic brackets.

### Detailed Description of Preferred Embodiments of the Invention

According to the plan, samples of this tooth movement activating compound were prepared with different viscosity levels, using refined virgin olive oil as a base.

Variable amounts of polyoxyethylene-20 sorbitan monooleate, among others, are used as a stabilising or mixing agent.

Other substances which complete the qualities of this tooth movement activating compound have also been added.

The process carried out consisted of preparing lipophilic creams, emulsions in oil, the creamy base thereof being a food or edible oil, which acts as an emulsifier. Another requirement is that they were stable over time and allowed the incorporation of all the predetermined reagents and furthermore complied with the sanitary-hygienic and food conditions in force.

The order of the mixture was a determining factor in the formation of the emulsion.

Component A: Mixture of aloe vera and polyoxyethylene-20 sorbitan monooleate.

Component B: Food oil.

Component A is introduced in a 400 ml beaker and stirred mechanically at room temperature until the formation of a homogeneous foam, component B is slowly added to this mixture. Once the addition has ended, the emulsion is taken to a thermostated bath at 45°C and the reagents NaF and triclosan are added, maintaining the stirring for 30 minutes. After that time, the system is taken out of the bath and, without stopping the stirring, allantoin and the corresponding additive are dispersed. The stirring is maintained until room temperature is reached.

Various formulations with different viscosity values according to the percentage of polysorbate-80 have been made. The percentages are by weight.

First preferred embodiment:
Food oil: 76.05 %
Aloe vera: 19.01 %
Stabilising or mixing agent: 3.80%
Additives:
   Triclosan: 0.27 %
   Sodium fluoride: 0.27 %
   98% Maltylisobutyrate: 0.34%.

A polyglycosylated saturated hydrogenated palm oil glyceride has been used as a stabilising or mixing agent in this and the following embodiments.

Second preferred embodiment:
Oil: 73.26%
Aloe vera: 18.32%
Stabilising or mixing agent: 7.30%
Additives:
   Triclosan: 0.26%
   NaF: 0.26%
   Allantoin: 0.26%
   99% D,L-menthol: 0.33%
Third preferred embodiment:
Oil: 71.94%
Aloe vera: 17.99%
Stabilising or mixing agent: 8.99%
Additives:
   Triclosan: 0.25%
   NaF: 0.25%
   Allantoin: 0.25%
   98% Maltylisobutyrate: 0.32%
Fourth preferred embodiment:
Oil: 77.52%
Aloe vera: 19.38%
Stabilising or mixing agent: 1.94%
Additives:
   Triclosan: 0.27%
   NaF: 0.27%
   Allantoin: 0.27%
   99% D,L-menthol: 0.35%
Fifth preferred embodiment:
Oil: 74.63%
Aloe vera: 18.66%
Stabilising or mixing agent: 5.60%
Additives:
   Triclosan: 0.26%
   NaF: 0.26%
   Allantoin: 0.26%
   99% D,L-menthol: 0.34%
Sixth preferred embodiment:
Oil: 71.94%
Aloe vera: 17.99%
Stabilising or mixing agent: 8.99%
Additives:
   Triclosan: 0.25%
   NaF: 0.25%
   Allantoin: 0.25% 98% Maltysobutyrate: 0.32%

In addition, samples with a higher consistency have been prepared by means of a different process, which consisted of slowly adding the stabilising or mixing agent to a two-phase system, subjected to mechanical stirring, which contained the remaining components, until the formation of a cream with consistency.

## Claims

1. A Tooth movement activating compound for use in orthodontics, **characterised in that** it comprises:
from 10% to 95% of a food type oil or fat which acts as a base.
from 5% to 70% of an aloe vera type antiseptic compound.
from 2% to 70% of a polyglycosylated saturated hydrogenated palm oil glyceride type stabilising or mixing agent.
from 0.05% to 2% of a triclosan type antibacterial and fungicidal compound.
from 0.05% to 2% of a sodium fluoride NaF type enamel protector.
from 0.05% to 2% of an allantoin type anti-inflammatory agent.

2. A Tooth movement activating compound for use in orthodontics according to claim 1, **characterised in that** the food type oil or fat, used as a base, is selected from the group consisting of fatty acid glycerides and are of a plant, animal (including milk) or marine origin such as castor oil, coconut oil, cottonseed oil, fish oil, grape seed oil, peanut oil, hydrogenated oils, linseed oil, corn oil, olive oil, olive pomace oil, palm oil, palm olein, palm stearin, palm kernel oil, rapeseed oil, safflower oil, sesame oil, sunflower oil, soybean oil, apricot kernel oil, babassu oil, mustard seed oil, turnip rape or ravison oil, lard, illipe butter, shea butter, tallow, synthetic edible fats or oils, liquid petroleum jelly, glycerol.

3. A tooth movement activating compound for use in orthodontics according to claim 1, **characterised in that** the additives are selected from the following groups; antiseptic agents such as triclosan, chlorophenylbiguanidine and derivatives, such as chlorhexidine, chlorhexidine digluconate, benzalkonium chloride, povidone-iodine molecular complex, mercurial antiseptic, merbromin, cetylpyridinium chloride, mouthwashes with hexidine, choline hexasalicylate, cetalkonium chloride, calcium carbonate, sodium citrate, dimethicone, nicomethanol hydrofluoride, 2^{nd} generation fluoroamines. Bactericides, for germs and bacteriostatic agents, potassium nitrate, xylitol, preparations with benzydamine HCl, chlorhexidine digluconate, hexetidine, Oralkin hexetidine. Fungicides such as, in addition to some of those mentioned above which have this property, sodium hypochlorite, acetic acid, alkaline peroxides, chlorhexidine gluconate. Antiseptic agents, ethanol, propanol, benzoic chloride. Antiviral agents, aloe vera, antiprotozoal agents, sporicides, anti-inflammatory agents, mucosal protectors, flavouring agents, antiplaque agents, such as zinc acetate, cetylpyridinium chloride, zinc lactate, fluorine derivatives such as sodium fluoride, sodium monofluorophosphate. Antihalitosis agents, such as zinc chloride, cetylpyridinium chloride, zinc lactate. Anticavity agents.
Most of these substances usually have more than one of the mentioned properties.

4. A tooth movement activating compound for use in orthodontics according to claim 1, **characterised in that** the stabilising or mixing compound is selected from the group consisting of polyglycosylated saturated hydrogenated palm oil glycerides, polyethylene glycol esters of apricot kernel oils or polyethylene glycol stearates.

5. Formulation process.
A process for manufacturing a tooth movement activating compound for use in orthodontics, **characterised in that** it comprises the following steps:
Component A: Mixture of aloe vera and polyoxyethylene-20 sorbitan monooleate.
Component B: Oil.
Formation of stable lipophilic creams with low to medium viscosity by slowly adding component B to a homogeneous foam of component A, which is previously obtained by means of a suitably stirring, thermostating at 45°C and after thirty minutes, incorporating the other reagents, finally, during the cooling process, allantoin is dispersed and the additives are added.

6. Formulation process.
A process for manufacturing a tooth movement activating compound for use in orthodontics, **characterised in that** it comprises the following steps:
Component A': Two-phase mixture of oil, aloe vera, triclosan, NaF, allantoin and corresponding additives.
Component B': Stabilising or mixing agent.
Formation of a stable cream with consistency by slowly adding component B' to the two-phase system, component A', under mechanical stirring and at room temperature.
